# FASCICULE DE BREVET EUROPEEN

(11) **EP 3 193 808 B1**
(45) Date de publication et mention de la délivrance du brevet: **27.11.2019**
(21) Numéro de dépôt: 15762646.6
(22) Date de dépôt: 15.09.2015
(51) Int. Cl.: A61G 9/00, A61G 7/057, A61B 10/00, A47K 13/08

(54) **ARTICLE POUR RECUEILLIR L'URINE ET LES SELLES D'UN UTILISATEUR**
ARTIKEL ZUR AUFNAHME VON URIN UND STUHL EINES BENUTZERS
ARTICLE FOR COLLECTING THE URINE AND STOOLS OF A USER

(30) Priorité: 15.09.2014 FR 1458658
(43) Date de publication de la demande: 26.07.2017
(73) Titulaire: Swiss Safe Collect SA, 2000 Neuchâtel (CH)
(72) Inventeur: CAILLETEAU, Benoit, 1807 Bionay (CH)
(74) Mandataire: Intès, Didier Gérard André
(86) Numéro de dépôt international: PCT/EP2015/071025
(87) Numéro de publication internationale: WO 2016/041927

(56) Documents cités:
- EP-A1- 0 658 337
- WO-A1-01/93738
- US-A- 5 394 571
- US-A1- 2003 116 575

## Description

### Arrière-plan de l'invention

La présente invention concerne un article pour recueillir l'urine et les selles d'un utilisateur, plus particulièrement d'un utilisateur alité, qu'il s'agisse d'un adulte ou d'un enfant, et qu'il soit de sexe féminin ou masculin.

Habituellement, pour recueillir l'urine et les selles d'un patient alité (notamment dans un hôpital ou une maison de retraite) qui n'est pas en mesure d'utiliser des toilettes, on emploie ou bien des couches jetables, ou bien des bassins lavables.

Aucune de ces solutions connues n'est satisfaisante du point de vue de l'hygiène.

En effet, lorsqu'une couche est usagée, elle doit être retirée du corps du patient pour être jetée puis remplacée par une autre couche propre. Il existe un risque de contact accidentel entre les matières fécales du patient et la personne (par exemple un infirmier) chargée de changer la couche, ou entre les matières fécales et un matériel quelconque, ou le sol.

Lorsqu'on utilise un bassin, celui-ci, n'étant pas jetable, doit être nettoyé pour être réutilisé. Cela signifie qu'à chaque fois que le bassin est souillé, une personne (par exemple un infirmier) doit intervenir pour retirer le bassin souillé, le remplacer par un autre bassin propre, puis transporter le bassin souillé vers un local technique où il peut être nettoyé puis désinfecté. Par conséquent, il y a de très nombreuses manipulations des bassins. De plus, le nettoyage et la désinfection des bassins peuvent être imparfaits, et un bassin imparfaitement désinfecté peut donc être remis en contact avec un autre patient, une autre personne, un autre matériel, ou le sol.

Or, il est maintenant connu que les contacts répétés avec les matières fécales, ou avec une surface ou un matériel contaminés par celles-ci, accroissent le risque de contamination par divers micro-organismes, notamment des bactéries, contenues dans les matières fécales. Cela est particulièrement problématique dans un établissement tel qu'un hôpital ou une maison de retraite, où les infections nosocomiales, en particulier par des bactéries multi-résistantes aux antibiotiques, représentent un risque pour la santé des patients, ainsi que pour celle du personnel soignant.

Il existe donc un besoin d'un dispositif pour recueillir l'urine et les selles d'un utilisateur qui permette de limiter ou d'éviter le contact accidentel entre les matières fécales d'un utilisateur et la personne chargée de manipuler le dispositif.

À ce titre, un conteneur jetable prévu pour s'adapter sur un bassin hygiénique classique a été proposé dans le document US 2003/0116575, qui divulgue ainsi un article selon le préambule de la revendication 1. Ce conteneur jetable comprend un compartiment inférieur de stockage pour stocker l'urine et les selles du patient, et un compartiment supérieur de réception pour diriger l'urine et les selles du patient vers le compartiment de stockage. Le compartiment de réception et le compartiment de stockage sont séparés entre eux par un élément anti-projections comprenant au moins un volet flexible. Le au moins un volet flexible délimite une fente qui s'ouvre pour laisser passer l'urine et les selles du patient, puis se referme une fois qu'elles sont passées à travers les selles.

Cependant, le conteneur jetable proposé dans le document US 2003/0116575 ne permet pas de séparer les selles de l'urine, même temporairement, ce qui augmente notamment le risque de souillure pour le patient. De plus, le document US 2003/0116575 ne propose pas de solution pour limiter ou empêcher l'apparition d'escarres lorsque l'utilisateur doit être alité pour une longue durée.

### Objet et résumé de l'invention

Pour remédier aux inconvénients ci-dessus, la présente invention propose un article pour recueillir l'urine et les selles d'un utilisateur, comprenant un bord de soutien pour supporter le fessier de l'utilisateur, et une chambre, qui communique avec une ouverture délimitée par le bord de soutien et qui présente une partie de fond, et un moyen d'écartement apte à maintenir la partie de fond à distance du bord de soutien, dans lequel la chambre comprend un élément de paroi interne, qui est situé à une position verticalement intermédiaire entre le bord de soutien et la partie de fond et qui est apte à adopter une première configuration et une deuxième configuration. Dans la première configuration, l'élément de paroi interne divise la chambre en un compartiment supérieur et un compartiment inférieur, en ménageant un obstacle entre ces compartiments pour retenir les selles dans le compartiment supérieur tout en permettant leur communication pour l'urine. Dans la deuxième configuration de l'élément de paroi interne, l'obstacle est sensiblement effacé de manière à laisser passer les selles vers le fond de la chambre.

Avec l'article décrit ci-dessus, puisque l'élément de paroi interne est situé à une position verticalement intermédiaire entre le bord de soutien et la partie de fond, lorsque l'élément de paroi interne est dans sa première configuration, le compartiment supérieur possède un volume intérieur tel qu'il permet de recevoir les selles de l'utilisateur installé sur l'article, qui peut ainsi uriner et déféquer dans l'article sans souiller d'autres matériels tels qu'un lit.

De plus, lorsque l'élément de paroi interne est dans sa première configuration, l'urine passe directement dans le compartiment inférieur, et les selles sont retenues par l'obstacle dans le compartiment supérieur tant que l'élément de paroi interne reste dans sa première configuration. Ainsi, les selles ne sont pas diluées dans l'urine, ce qui limite les risques de souillure de l'utilisateur d'autant que, l'article de l'invention étant plutôt utilisé pour des personnes malades, assises ou alitées, il reste en général un certain temps au contact de l'utilisateur, sur son siège ou dans son lit.

Ensuite, lorsqu'on désire remplacer l'article souillé par un autre article propre, la personne chargée de remplacer l'article provoque le passage de l'élément de paroi interne de sa première configuration à sa deuxième configuration. Dans la deuxième configuration de l'élément de paroi interne, l'obstacle formé par l'élément de paroi interne est sensiblement effacé, c'est-à-dire que l'élément de paroi interne demeure à l'intérieur de la chambre, mais que sa conformation est modifiée de telle sorte qu'il ne fasse sensiblement plus obstacle au passage des selles. Les selles passent alors vers le compartiment inférieur et y sont piégées. De cette manière, on éloigne davantage les selles de l'utilisateur et de la personne chargée de remplacer l'article. Ainsi, le risque de contact avec les matières fécales est éliminé ou grandement limité.

L'élément de paroi interne comprend deux parties de film souple.

Ainsi, la construction de l'élément de paroi interne est relativement simple, car il suffit de solidariser chacune des deux parties de film souple avec une paroi de la chambre.

L'obstacle est formé par deux plis rentrant des deux parties de film souple. Plus précisément, dans la première configuration, la communication entre les compartiments comprend une ouverture sensiblement en forme de fente, la fente étant délimitée par deux plis rentrants de deux parties de film souple placées l'un en face de l'autre, et l'obstacle étant formé par les deux plis rentrants.

Il est ainsi particulièrement aisé d'effacer l'obstacle lors du passage de la première à la deuxième configuration, puisque cet effacement peut s'opérer par un simple dépliage des plis. Par ailleurs, les plis renforcent localement la partie de film souple correspondante qui, dans la première configuration, reste bien positionnée en évitant un effacement intempestif de l'obstacle, la portion pliée formant une séparation efficace entre les deux compartiments et un support efficace pour les selles, dans la première configuration. De plus, lors du passage de la première configuration à la deuxième configuration, l'effacement de l'obstacle s'effectue de concert avec l'élargissement de la fente.

La fente peut être sensiblement dans la direction longitudinale de l'article ou sensiblement dans la direction transversale de l'article.

Selon une possibilité, dans la première configuration, la largeur de la fente est comprise entre 0,5 cm et 3 cm.

Cette largeur est alors suffisante pour laisser passer l'urine tout en retenant substantiellement les selles de l'utilisateur.

Selon une possibilité, la longueur de la fente est comprise entre 10 cm et 60 cm.

Cette longueur est alors suffisante pour permettre le passage de la quasi-totalité des selles de l'utilisateur lors du passage de la première configuration à la deuxième configuration.

Les deux parties de film souple sont reliées à leur extrémité supérieure au bord de soutien et à leur extrémité inférieure à la partie de fond.

Ainsi, pour provoquer le passage de l'élément de paroi interne de sa première à sa deuxième configuration, il suffit de saisir la partie de l'article portant le bord de soutien et de tirer cette partie vers le haut, ce qui provoque automatiquement le dépliage des plis rentrants. La manipulation de l'article est alors simplifiée, et, si l'article est manipulé correctement, le risque de contact accidentel entre la personne chargée de manipuler l'article et les selles du patient est très limité.

Selon une possibilité, une partie de la chambre s'étend jusque sous le bord de soutien.

Ainsi, la contenance de la chambre est accrue.

Selon une possibilité, un élément absorbant est disposé sur la partie de fond.

Ainsi, on limite le risque que de l'urine se répande lorsque l'article est accidentellement renversé, ou lorsque que le fond est percé, ou lorsque l'élément de paroi est endommagé. De plus, on évite ou élimine le risque d'éclaboussures lorsque les selles passent brusquement du compartiment supérieur vers le compartiment inférieur, lorsque ce dernier a auparavant recueilli une quantité importante d'urine.

Selon une possibilité, le moyen d'écartement est un boudin gonflable.

Ainsi, il est possible de maintenir la partie de fond à distance du bord de soutien à l'aide d'un moyen simple d'utilisation.

Selon une possibilité, le bord de soutien est sur la surface supérieure du boudin gonflable.

De cette manière, le fessier de l'utilisateur est soutenu par la partie gonflable. L'article peut alors servir à la fois à recueillir l'urine et les selles d'un utilisateur alité, mais aussi à limiter ou empêcher l'apparition d'escarres lorsque l'utilisateur doit être alité pour une longue durée.

Selon une possibilité, le boudin gonflable comprend une valve de gonflage munie d'un clapet anti-retour.

Ainsi, le boudin gonflable peut être gonflé par un apport d'air extérieur sous pression, sans se dégonfler de manière indésirable.

Selon une possibilité, la plus grande dimension de l'ouverture délimitée par le bord de soutien est au moins égale à 20 cm.

La taille de l'ouverture est alors suffisante pour sensiblement entourer la région génitale de l'utilisateur, ce qui évite que d'autres matériels, tels qu'un lit, soient souillés par de l'urine ou des selles.

### Brève description des dessins

L'invention sera bien comprise et ses avantages apparaîtront mieux, à la lecture de la description détaillée qui suit de plusieurs modes de réalisations, représentés à titre d'exemples non limitatifs. La description se réfère aux dessins annexés sur lesquels :
- la figure 1 est une vue en perspective de dessus d'un article selon un premier mode de réalisation de l'invention ;
- la figure 2 est une coupe selon II-II de l'article représenté sur la figure 1 ;
- les figures 3A et 3B sont des coupes selon II-II de l'article représenté sur la figure 1, lorsque celui-ci est en cours d'utilisation ;
- la figure 4A est une vue de dessus de l'article représenté sur la figure 1 ;
- la figure 4B est une vue analogue à la figure 4A, pour une variante de réalisation ;
- la figure 5 est une vue en perspective de dessus de l'article selon un second mode de réalisation de l'invention ;
- la figure 6 est une coupe selon VI-VI de l'article représenté sur la figure 5 ;
- les figures 7A et 7B sont des coupes selon VI-VI de l'article représenté sur la figure 5, lorsque celui-ci est en cours d'utilisation ;
- la figure 8A est une vue de dessus de l'article représenté sur la figure 5 ;
- la figure 8B est une vue analogue à la figure 8A, pour une variante de réalisation.

### Description détaillée de l'invention

Sur toutes les figures et dans l'exposé qui suit, Dv, Dℓ et Dt désignent respectivement les directions verticale, longitudinale et transversale de l'article selon l'invention.

La direction verticale Dv est la direction perpendiculaire à la surface (par exemple, celle d'un lit) sur laquelle est installé l'article. La direction longitudinale Dℓ de l'article est celle qui coïncide avec l'axe tête-torse-jambes d'un utilisateur lorsqu'il est installé sur l'article.

La direction transversale Dt est la direction perpendiculaire, dans un plan horizontal, à la direction longitudinale Dℓ.

### Premier mode de réalisation

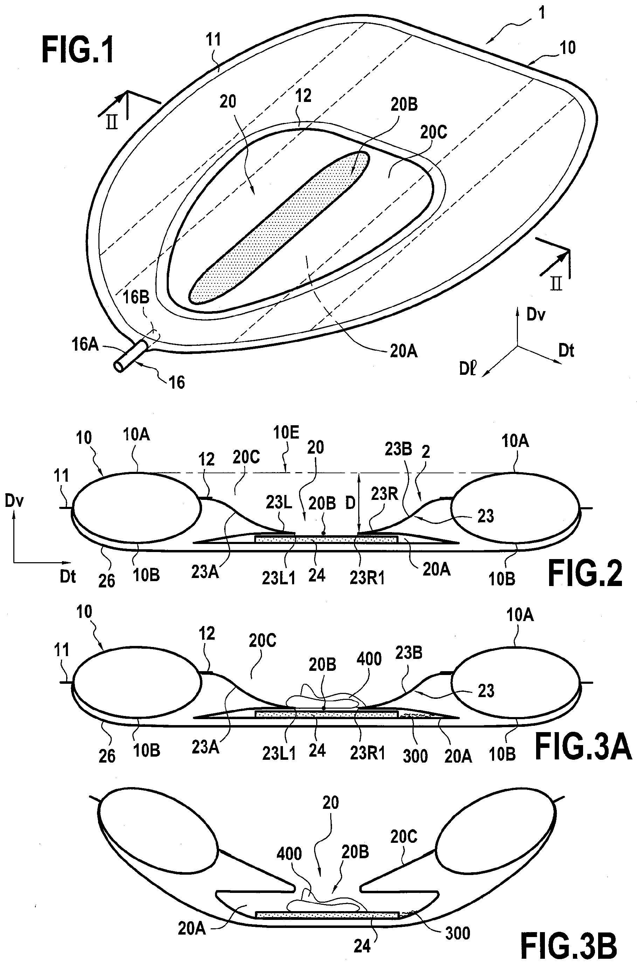

Comme on le voit sur la figure 1, un article 1 selon un premier mode de réalisation comprend un boudin gonflable 10 délimitant une ouverture 20B.

Le boudin gonflable 10 est réalisé en reliant deux films découpés aux formes (en particulier, en anneau) et dimensions appropriées de manière à obtenir un bord de liaison extérieur 11 et un bord de liaison intérieur 12. Par exemple, les films sont des films minces de matière plastique (par exemple du polyéthylène (PE)) que l'on déroule en continu, la liaison entre eux étant réalisée par fusion locale de la matière plastique comme cela est bien connu. La matière plastique peut aussi être un bioplastique, en particulier un plastique biodégradable, voire compostable, par exemple à base d'amidon de maïs. Bien entendu, la liaison entre les films peut aussi s'effectuer par un autre moyen que la fusion locale de la matière plastique, par exemple par collage.

Le boudin gonflable 10 présente un bord supérieur 10A, qui sert de bord de soutien pour le corps d'un utilisateur comme cela sera décrit en détail plus loin, et un bord inférieur 10B. Le bord supérieur 10A délimite une ouverture 10E en son centre.

Le boudin gonflable 10 présente une valve de gonflage 16 comprenant un tuyau d'admission 16A et un clapet anti-retour 16B. La valve de gonflage 16 peut être positionnée n'importe où sur le boudin gonflable 10.

Comme cela sera détaillé plus loin, la valve de gonflage 16 est utilisée pour gonfler le boudin gonflable 10 avant d'installer l'article 1 sous un utilisateur. Le boudin gonflable 10 peut être gonflé par n'importe quel moyen, y compris en faisant souffler une personne dedans comme on le ferait dans un ballon de baudruche. Toutefois, on peut également utiliser une pompe extérieure, une pompe installée sur l'article 1 (non représentée), ou de l'air ou de l'oxygène médical sous pression lorsqu'il est disponible dans un établissement tel qu'un hôpital ou une maison de retraite. Le clapet anti-retour 16B sert, lorsque le boudin gonflable 10 est gonflé ou en cours de gonflage, à éviter que ce dernier ne se dégonfle. Des clapets anti-retour adaptés pour cela sont connus et ne seront pas décrits en détail ici.

On notera que, en complément ou en remplacement d'un gonflage par de l'air ou de l'oxygène extérieurs, on pourrait aussi placer dans le boudin gonflable 10 une quantité appropriée de composés chimiques qui possèdent la propriété de produire un gaz lorsqu'ils sont mis en contact, et prévoir un système de mise en contact, par exemple par rupture d'une paroi interne séparant initialement ces composés.

Comme on le voit sur la vue en coupe de la figure 2, l'article 1 présente une chambre 20 qui comprend un élément de paroi interne 23.

L'élément de paroi interne 23 est situé à une position verticalement intermédiaire entre le bord de soutien 10A et le fond 22 de l'article 1. En d'autres termes, lorsque l'article 1 est posé à l'horizontale sur une surface, l'élément de paroi interne 23 est situé plus bas que le bord de soutien 10A et plus haut que le fond 22. Dans la mesure où il peut être flexible, l'élément de paroi interne 23 peut toutefois, sous l'effet de son propre poids, tendre à toucher le fond.

La figure 2 représente l'article 1 lorsque l'élément de paroi interne 23 est dans la configuration qu'il adopte lorsque le boudin gonflable 10 est gonflé et l'article 1 est posé au repos sur une surface horizontale (ci-après « première configuration »).

Dans cette première configuration, l'élément de paroi interne 23 divise la chambre 20 en un compartiment supérieur 20C et un compartiment inférieur 20A.

Le compartiment supérieur 20C communique en haut avec l'extérieur par l'ouverture 10E, et est délimité latéralement par les bords intérieurs du boudin gonflable 10.

Puisque l'élément de paroi interne 23 est situé plus bas que le bord de soutien 10A, on comprend que le compartiment supérieur 20C possède des dimensions verticales et un volume intérieur tels qu'il peut recevoir les selles 400 de l'utilisateur (voir figure 3A). De préférence, on choisit la hauteur de l'élément de paroi interne 23 de telle sorte que, lorsque l'utilisateur a déféqué, les selles 400 soient maintenues à distance de la peau de l'utilisateur. Par exemple, la distance D entre l'élément de paroi interne et le plan défini par le bord de soutien, est de l'ordre de 3 à 10 cm.

Le compartiment inférieur 20A est situé sous le compartiment supérieur 20C et communique avec lui via une ouverture 20B délimitée par l'élément de paroi interne 23. Le fond du compartiment inférieur 20A coïncide avec le fond 22 de l'article 1.

De manière générale, les dimensions verticales du compartiment inférieur 20A sont faibles (sur les figures 2 et 3A, on les a exagérées pour des raisons de clarté), et sa paroi supérieure, définies en l'espèce par la face inférieure de l'élément de paroi interne, peuvent même entrer en contact avec le fond 22, sans que cela ne nuise pour autant au fonctionnement de l'article 1. Toutefois, il est souhaitable que le compartiment inférieur 20A puisse contenir l'urine 300 de l'utilisateur. Pour cela, dans l'exemple représenté, le fond 22 du compartiment est garni par un élément absorbant 24 qui, quitte à subir une légère augmentation de volume, peut absorber l'urine. L'élément absorbant 24 peut être du type de ceux classiquement utilisés dans les couches absorbantes pour enfants ou adultes. Il peut être fixé (par exemple par collage) sur la partie de fond 22, ou alors simplement posé sur la partie de fond 22.

Bien que cela ne soit pas représenté sur les figures, si on désire augmenter la contenance du compartiment inférieur 20A, on peut agrandir celui-ci dans la direction transversale Dt de sorte qu'il s'étende partiellement jusque sous le bord supérieur 10A.

Dans la première configuration (voir figure 3A), l'élément de paroi interne 23 forme un obstacle au passage des selles 400 de l'utilisateur. Par « former un obstacle », on entend que l'élément de paroi interne 23 tend à retenir les selles 400, en empêchant ou limitant leur passage vers le fond de la chambre 20, et que par conséquent, les selles 400 sont essentiellement maintenues à distance de la partie de fond 22, sans rester au contact de l'urine contenue dans le compartiment inférieur 20A.

On prévoit également que l'élément de paroi interne puisse adopter, suite à une manipulation extérieure, une configuration différente de la première configuration (ci-après « deuxième configuration »).

Dans la deuxième configuration, l'obstacle formé par l'élément de paroi interne 23 est sensiblement effacé, c'est-à-dire que l'élément de paroi interne 23 demeure à l'intérieur de la chambre 20, mais que sa conformation est modifiée de telle sorte qu'il ne fasse sensiblement plus obstacle au passage des selles 400 (par exemple parce que l'ouverture 20B s'est élargie). Les selles 400 passent alors par gravité vers le fond de la chambre 20. Ainsi, les selles 400 et l'urine 300 se retrouvent ensemble au fond de la chambre 20 et à distance de l'ouverture 10E, du bord de soutien 10A et de la peau de l'utilisateur, de sorte que les risques de contact entre les selles 400 et la peau de la personne qui manipule l'article dans sa deuxième configuration sont limités.

De manière facultative, l'article 1 peut comprendre une paroi extérieure 26, par exemple un film souple en polyéthylène (PE) ou en plastique biodégradable, relié au bord de liaison extérieur 11, englobant l'élément de paroi interne 23 et doublant la partie de fond 22, c'est-à-dire que si la partie de fond 22 est accidentellement endommagée, de l'urine ou des selles qui s'en échappent sont piégés par la paroi extérieure 26.

On va maintenant décrire l'utilisation et le fonctionnement de l'article 1.

Pour installer l'article 1 entre une surface, par exemple celle d'un lit où un utilisateur est alité, et le corps de l'utilisateur, on gonfle le boudin gonflable 10 comme cela a été décrit ci-dessus, puis on installe l'utilisateur de sorte que son fessier soit soutenu par le bord de soutien 10A. Ainsi, lorsque l'utilisateur est alité, le boudin gonflable 10 soutient l'utilisateur, ce qui limite ou empêche l'apparition d'escarres. On notera que, lorsque l'utilisateur est installé, l'élément de paroi interne 23 est dans la première configuration mentionnée ci-dessus.

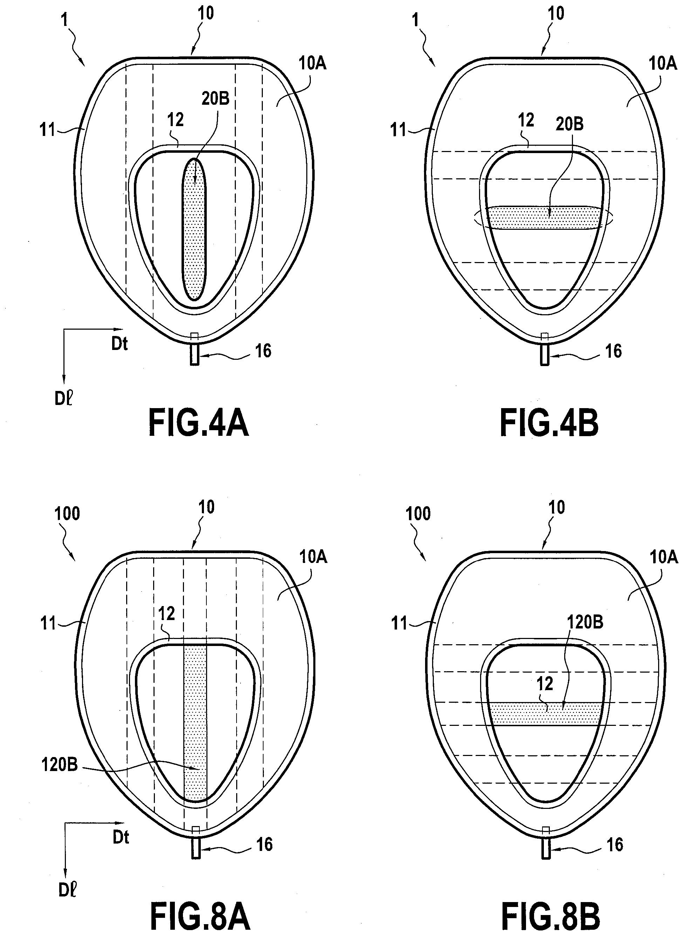

Comme on le voit sur les figures 1, 4A et 4B, le contour extérieur du boudin gonflable 10 a de préférence une forme généralement ovoïde

(c'est-à-dire une forme analogue au contour d'un œuf), de telle sorte que sa grande dimension coïncide avec la direction longitudinale Dℓ, sa petite dimension coïncide avec la direction transversale Dt, et que le contour soit plus large dans la région du boudin gonflable 10 qui est proche du torse de l'utilisateur (en haut sur les figures 4A et 4B). Ceci améliore le soutien procuré à l'utilisateur.

Pour améliorer encore le soutien procuré à l'utilisateur, on peut également prévoir que le boudin soit plus large et/ou plus épais dans sa région proche du dos de l'utilisateur (en haut sur les figures 4A et 4B). Toutefois, on peut envisager d'autres formes pour le boudin gonflable 10 si nécessaire.

En tout état de cause, on installe l'utilisateur de telle sorte que son axe tête-torse-jambes coïncide sensiblement avec la direction longitudinale Dℓ, et que sa région génitale soit au-dessus de l'ouverture 10E du boudin gonflable et de l'ouverture 20B.

En résumé, l'article 1 ressemble généralement à une bouée, de préférence de forme ovoïde, à laquelle on a ajouté une chambre 20 pour recevoir de l'urine ou des selles, et l'utilisateur est allongé de sorte que sa région génitale soit au-dessus de l'ouverture centrale de la bouée.

Après que l'utilisateur a uriné et/ou déféqué, il devient nécessaire de remplacer l'article 1.

Lorsque l'élément de paroi interne 23 est dans la première configuration, il forme un obstacle au passage des selles 400. Comme cela a été expliqué ci-dessus, les selles 400 sont ainsi maintenues à distance de la peau de l'utilisateur. D'autre part, comme l'ouverture 20B n'est pas fermée, elle ménage une communication à travers laquelle l'urine 300 peut passer vers le compartiment inférieur 20A.

Par une manipulation extérieure de l'article 1, on provoque le passage de la première configuration à la deuxième configuration de l'élément de paroi interne 23. Ainsi, l'obstacle formé par l'élément de paroi interne 23 s'efface sensiblement, ce qui permet aux selles 400 de passer vers le compartiment inférieur 20A.

Si un élément absorbant 24 a été disposé sur la partie de fond 22, la majeure partie ou la totalité de l'urine 300 est absorbée par l'élément absorbant 24. Ainsi, on évite ou élimine le risque d'éclaboussures lorsque les selles 400 passent trop brusquement vers le compartiment inférieur 20A, lorsque celui-ci a auparavant recueilli une quantité importante d'urine 300. On évite aussi que de l'urine ne se répande lorsque l'article est accidentellement renversé, ou lorsque que la partie de fond 22 est percée, ou plus généralement lorsque le film souple 23 est endommagé. Ces deux derniers points sont particulièrement désirables lorsque, pour des raisons d'économie, on choisit de ne pas doubler le film souple 23 par la paroi extérieure 26 comme cela a été décrit plus haut.

Lorsque l'élément de paroi interne 23 est dans sa deuxième configuration, on peut transporter l'article 1 souillé vers un autre lieu, et le remplacer par un nouvel article 1 propre.

De manière générale, on envisage que l'article 1 soit à usage unique et jetable. Cela signifie que l'article 1 est prévu pour être directement éliminé après utilisation, par exemple par incinération. À cet égard, la construction en films souples de polyéthylène (PE) ou de bioplastique est avantageuse, car l'article 1 a ainsi un coût suffisamment faible pour que cela soit économiquement viable. D'autre part, si l'article 1 est jetable, il n'est plus nécessaire de prévoir un lieu pour nettoyer les articles souillés, comme c'est actuellement le cas avec les bassins connus, ou de faire circuler les articles dans l'établissement. Ainsi, on évite la multiplication de contacts entre les articles et les autres matériels ou surfaces.

Concrètement, pour jeter l'article 1, on dégonfle le boudin gonflable 10, par exemple en le déchirant au niveau d'une ligne d'affaiblissement (non représentée) sur le boudin gonflable 10, ou alors en le perçant avec un objet pointu, puis on le place dans un conteneur approprié. Si les circonstances l'exigent, le conteneur peut être un conteneur pour recevoir des déchets médicaux.

On va maintenant décrire plus en détail une configuration possible de l'élément de paroi interne 23.

L'élément de paroi interne 23 peut comprendre au moins une partie de film souple qui peut être reliée à son extrémité supérieure au bord de soutien (plus précisément, au boudin gonflable 10) et à son extrémité inférieure à la partie de fond 22. On relève que la au moins une partie de film souple et la partie de fond peuvent être formés à partir d'un même film, convenablement plié.

Par exemple, l'extrémité supérieure de la au moins une partie de film souple peut être reliée au bord de liaison intérieur 12, ou plus généralement au bord inférieur 10B du boudin gonflable 10. Il n'est pas nécessaire que les parties de film souple soient reliées au boudin gonflable 10 de manière symétrique. En d'autres termes, les extrémités supérieures des parties de film souple peuvent être reliées au boudin gonflable 10 à des hauteurs différentes les unes des autres.

Dans ce mode de réalisation, l'ouverture 20B est fermée (par exemple par soudure locale) à ses extrémités proches du voisinage du bord de liaison intérieur 12 (voir figures 4A et 4B).

La au moins une partie de film souple est conformée de telle sorte que, dans la première configuration, elle forme au moins un pli rentrant, de sorte que l'obstacle au passage des selles 400 est formé par ce au moins un pli rentrant. Au sens de la présente description, le fait que le pli soit « rentrant » signifie que la charnière du pli (repérée par les références 23L1 et 23R1 sur la figure 2) se situe davantage vers l'intérieur de l'article vu en coupe dans la direction transversale Dt que les deux flancs du pli séparés par la charnière. En d'autres termes, la forme du pli tend à projeter la au moins une partie de film souple vers l'intérieur de la chambre 20.

Comme pour le boudin gonflable 10, la au moins une partie de film souple peut comprendre un film souple en polyéthylène (PE) ou en plastique biodégradable. De préférence, la au moins une partie de film souple a une épaisseur suffisante pour supporter le poids des selles 400 sans être déchirée ou endommagée, mais suffisamment faible pour qu'elle puisse se plier et se déplier aisément. Par exemple, cette épaisseur est comprise entre 20 µm et 500 µm, de préférence entre 30 µm et 100 µm.

Les figures 2, 3A et 3B illustrent une configuration d'exemple dans laquelle l'article 1 comprend deux parties de film souple 23A et 23B placées l'un en face de l'autre. Les parties de film souple 23A et 23B comportent chacune un pli rentrant, désignés respectivement par 23L et 23R. Les charnières 23L1 et 23R1 de ces plis rentrants délimitent l'ouverture 20B, qui a alors substantiellement une forme de fente.

Comme cela est visible sur les figures 4A et 4B, la fente peut être sensiblement alignée avec la direction longitudinale Dℓ, ou sensiblement alignée avec la direction transversale Dt. Le fonctionnement de l'article 1 est identique dans les deux cas de figure.

Afin de permettre le passage de la quasi-totalité des selles de l'utilisateur lors du passage de la première configuration à la deuxième configuration comme cela sera décrit ci-dessous, la fente peut avoir une longueur comprise entre 10 cm et 60 cm. Toutefois, pour ce mode de réalisation, il est préférable que la longueur de la fente soit comprise entre 10 cm et 40 cm.

De préférence, afin que la taille de l'ouverture 10E soit alors suffisante pour sensiblement entourer la région génitale de l'utilisateur, ce qui évite que d'autres matériels tels qu'un lit soient souillés par de l'urine ou des selles, on choisit l'ouverture 10E de telle sorte que sa plus grande dimension soit au moins égale à 20 cm.

Dans la première configuration de l'élément de paroi interne 23, représentée sur la figure 3A, les plis rentrants 23L et 23R forment un obstacle au passage des selles 400. Cependant, comme la fente 20B délimitée par les charnières 23L1 et 23R1 des plis rentrants 23L et 23R n'est pas fermée, elle ménage une communication à travers laquelle l'urine 400 peut passer vers compartiment inférieur 20A.

Par exemple, pour ménager une communication qui permette à l'urine 300 de passer sans laisser passer les selles 400, la fente 20B peut avoir une largeur comprise entre 0,5 cm et 3 cm.

Pour provoquer le passage de l'élément de paroi interne 23 de sa première configuration (figure 3A) à sa deuxième configuration (figure 3B), il suffit de saisir le boudin gonflable 10 en deux points distincts (de préférence sensiblement diamétralement opposés), de préférence situés sur sa périphérie extérieure, et de le soulever vers le haut dans la direction verticale Dv. Puisque les parties de film souple 23A et 23B sont fixées au bord inférieur 10B du boudin gonflable 10, ou au bord de liaison intérieur 12, les plis rentrants 23L et 23R « se déplient » progressivement à mesure que le boudin gonflable 10 est tiré vers le haut. On notera qu'il est préférable de saisir le boudin gonflable 10 dans une direction perpendiculaire à l'axe de la fente (c'est-à-dire que la ligne reliant les deux points de saisie est sensiblement perpendiculaire à l'axe de la fente), afin de ne pas gêner le dépliement du film souple 23A.

Lorsque les plis rentrants 23L et 23R « se déplient », la fente 20B s'élargit progressivement. Ainsi, l'obstacle formé par les plis rentrants 23L et 23R s'efface sensiblement, ce qui permet aux selles 400 de passer vers le compartiment inférieur 20A.

Bien que l'article qui vient d'être décrit présente un pli rentrant de chaque côté d'une ouverture substantiellement en forme de fente, on pourrait aussi prévoir une ouverture d'une forme différente (en triangle, en quadrilatère, etc.) avec une partie de film souple présentant un pli rentrant pour chaque côté de l'ouverture, ou bien des configurations où un ou plusieurs côtés de l'ouverture présentent deux (ou plus) plis superposés.

On comprend alors que si l'article 1 est correctement manipulé, le risque de contacts accidentels entre les selles 400 et la personne chargée de remplacer l'article 1 est très faible. En effet, puisque la personne doit seulement saisir deux points du boudin gonflable 10, elle n'est pas amenée à entrer en contact avec les selles 400, ou même avec un élément de l'article qui a été en contact avec les selles 400. D'autre part, lorsque l'on saisit l'article 1 en deux points sensiblement diamétralement opposés du boudin gonflable 10, on obtient une bonne préhension de l'article 1, et le risque de renversement accidentel est limité. Enfin, puisque les selles 400 sont piégées dans le compartiment inférieur 20A, le risque de contact accidentel entre les selles 400 et une autre surface, un autre matériel ou le sol est plus limité qu'avec une couche.

### Deuxième mode de réalisation

Dans ce qui suit, on va décrire le deuxième mode de réalisation en se concentrant sur les différences avec le premier mode de réalisation.

Ainsi, dans les figures relatives au deuxième mode de réalisation et dans l'exposé qui suit, les éléments du deuxième mode de réalisation qui sont identiques à ceux du premier mode de réalisation sont désignés par les mêmes numéros de référence, et ne sont mentionnés de nouveau que lorsque cela est nécessaire.

Les éléments du deuxième mode de réalisation qui diffèrent du premier mode de réalisation sont désignés par des numéros de référence supérieurs à 100.

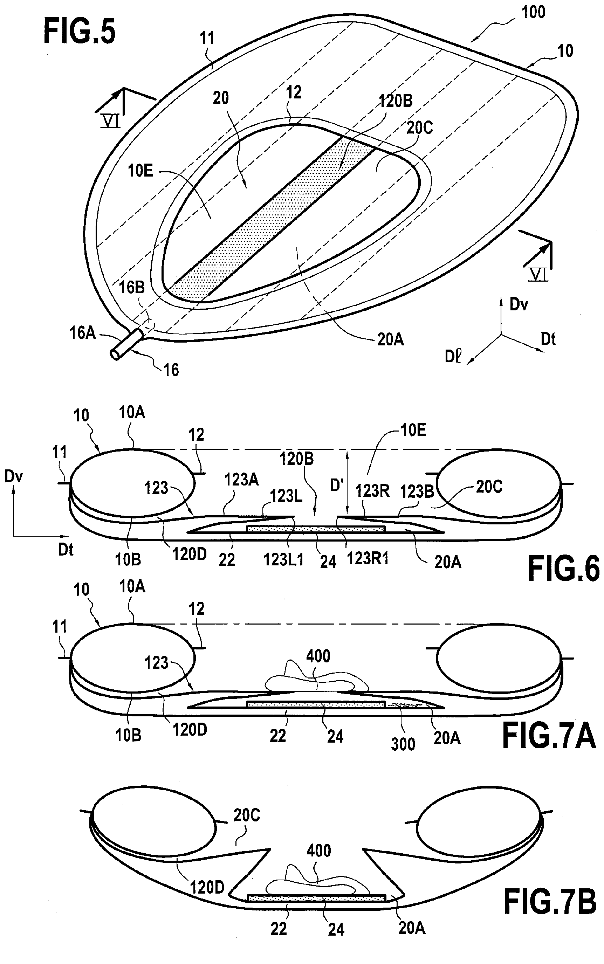

La figure 5 est une vue en perspective de dessus de l'article 100 selon le deuxième mode de réalisation. L'article 100 est généralement semblable à l'article 1 décrit ci-dessus, sauf en ce qui concerne l'élément de paroi.

Plus précisément, comme cela est mieux visible sur la figure 6, l'article comprend un élément de paroi interne 123 qui correspond à l'élément de paroi interne 23 du premier mode de réalisation, et comprend comme lui au moins une partie de un film souple. Ici encore, on a représenté deux parties de film souple 123A et 123B, sachant que ce qui suit peut être généralisé à un nombre quelconque de parties de film souple.

Dans ce mode de réalisation, les extrémités supérieures respectives des parties de film souple 123A et 123B sont fixées au bord de liaison extérieur 11 et non au bord de liaison intérieur 12 ou ailleurs sur le bord inférieur 10B du boudin gonflable 10. Ici encore, il n'est pas nécessaire que les extrémités supérieures des parties de film souple 123A et 123B soient reliées au boudin gonflable 10 de manière symétrique. Par exemple, si nécessaire, l'une des deux liaisons peut être faite au niveau du bord de liaison extérieur 12, et l'autre au niveau du bord de liaison intérieur 11 ou ailleurs sur le bord inférieur 10B.

Par conséquent, l'obstacle formé au passage des selles 400 se trouve légèrement plus bas que dans le premier mode de réalisation.

Comme dans le premier mode de réalisation, la chambre 20 est divisée, lorsque l'élément de paroi interne est dans sa première configuration, en un compartiment supérieur 20C et un compartiment inférieur 20A communiquant entre eux par une ouverture 120B.

Puisque les parties de film souple 123A et 123B sont fixées par leurs extrémités supérieures au bord de liaison extérieur 11, une partie périphérique 120D du compartiment supérieur 20C s'étend sous le bord de soutien 10A. Si on désire augmenter encore la contenance de la chambre 20, on peut aussi prévoir qu'une partie du compartiment inférieur 20A s'étende jusque sous le bord de soutien 10A.

Comme dans l'exemple correspondant du premier mode de réalisation, les parties de film souple 123A et 123B sont pliées de telle manière qu'elles comportent chacune un pli rentrant, respectivement désignés par 123L et 123R. Les charnières 123L1 et 123R1 de ces plis rentrants délimitent l'ouverture 120B, qui est substantiellement en forme de fente.

Comme cela est visible sur les figures 8A et 8B, la fente peut être sensiblement alignée avec la direction longitudinale Dℓ, ou sensiblement alignée avec la direction transversale Dt. Le fonctionnement de l'article est identique dans les deux cas.

Dans ce deuxième mode de réalisation, et contrairement au premier mode de réalisation, l'ouverture 120B n'est pas fermée au voisinage du bord de liaison intérieur 12, et s'étend au contraire jusque sous le bord de soutien 10A du boudin gonflable 10 (voir figures 8A et 8B).

On notera qu'ici aussi, pour simplifier la construction de l'article 1, on peut prévoir que la partie de fond 22 et les parties de film souple 123A et 123B soient formées à partir d'un seul film souple convenablement disposé et relié à ses extrémités au bord de liaison extérieur 12.

Afin de permettre le passage de la quasi-totalité des selles de l'utilisateur lors du passage de la première configuration à la deuxième configuration comme cela sera décrit ci-dessous, la fente peut avoir une longueur comprise entre 10 cm et 60 cm. Toutefois, pour ce mode de réalisation, il est préférable que la longueur de la fente soit comprise entre 40 cm et 60 cm.

Pour ménager une communication qui permette à l'urine 300 de passer sans laisser passer les selles 400, la fente 120B peut avoir une largeur comprise entre 0,5 cm et 3 cm.

De préférence, afin que la taille de l'ouverture 10E soit alors suffisante pour sensiblement entourer la région génitale de l'utilisateur, ce qui évite que d'autres matériels tels qu'un lit soient souillés par de l'urine ou des selles, on choisit l'ouverture 10E de telle sorte que sa plus grande dimension soit au moins égale à 20 cm.

Le fonctionnement de l'article 100 est représenté sur les figures 7A et 7B et est identique à celui du premier mode de réalisation : lorsque l'article 100 est souillé par de l'urine 300 et des selles 400, on provoque le passage de l'élément de paroi interne 123 de sa première configuration (figure 7A) à sa deuxième configuration (figure 7B), en saisissant le boudin gonflable 10 en deux points du boudin gonflable 10, puis en le soulevant vers le haut dans la direction verticale Dv.

De la même manière que dans le premier mode de réalisation, les plis rentrants 123L et 123R « se déplient » progressivement à mesure que le bord de liaison extérieur 11 est tiré vers le haut, la fente 120B s'élargit progressivement, et l'obstacle formé par les plis rentrants 123L et 123R s'efface sensiblement, ce qui permet aux selles 400 de passer vers le fond 22 du compartiment inférieur 20A. On obtient ainsi les mêmes avantages en termes d'hygiène qu'avec l'article 1 du premier mode de réalisation.

En comparaison avec l'article 1 du premier mode de réalisation, l'article 100 présente les avantages supplémentaires suivants.

Dans la première configuration de l'élément de paroi interne 123, l'obstacle formé au passage des selles 400 se trouve légèrement plus bas que dans le premier mode de réalisation. Par exemple, la distance D'entre l'élément de paroi interne et le plan défini par le bord de soutien, est de l'ordre de 4 à 10 cm. Ceci permet de limiter les contacts involontaires entre les selles 400 et la peau de l'utilisateur ou de la personne chargée de remplacer l'article 100.

De plus, lors du passage de la première configuration à la deuxième configuration, une pente se crée naturellement depuis la paroi du compartiment supérieur 20C vers la fente 120B. Ceci contribue à faire passer les selles vers le compartiment inférieur 20A. L'utilisation de l'article 100 se trouve donc facilitée. En particulier, par rapport au premier mode de réalisation, il y a moins de risques que des selles 400 se retrouvent coincées dans le compartiment supérieur 20C ou sur le bord de liaison intérieur 12.

Bien que les articles 1 et 100 ci-dessus aient été décrits dans un contexte où ils sont utilisés pour un utilisateur alité dans un établissement comme un hôpital ou une maison de retraite, il est bien évident que l'invention ne se limite pas à ce contexte précis. En particulier, l'article peut aussi être utilisé en position assise, et par conséquent, il n'est pas indispensable que l'utilisateur soit allongé sur l'article.

Quoique la présente invention ait été décrite en se référant à des exemples de réalisation spécifiques, il est évident que des modifications et changements peuvent être effectués sur ces exemples sans sortir de la portée générale de l'invention telle que définie par les revendications. En outre, des caractéristiques individuelles des différents modes de réalisation évoqués peuvent être combinées dans des modes de réalisation additionnels. Par conséquent, la description et les dessins doivent être considérés dans un sens illustratif plutôt que restrictif.

## Revendications

1. Article (1 ; 100) pour recueillir l'urine (300) et les selles (400) d'un utilisateur, comprenant :
un bord de soutien (10A) pour supporter le fessier de l'utilisateur ; et
une chambre (20), qui communique avec une ouverture (10E) délimitée par le bord de soutien (10A) et qui présente une partie de fond (22), et
un moyen d'écartement (10) apte à maintenir la partie de fond à distance du bord de soutien (10A) ;
la chambre comprenant un élément de paroi interne (23 ; 123), qui est situé à une position verticalement intermédiaire entre le bord de soutien (10A) et la partie de fond (22) et qui est apte à adopter une première configuration et une deuxième configuration, l'élément de paroi interne (23 ; 123) comprenant deux parties de film souple (23A, 23B ; 123A, 123B),
dans lequel, dans la première configuration, l'élément de paroi interne (23 ; 123) divise la chambre en un compartiment supérieur et un compartiment inférieur, en ménageant un obstacle entre ces compartiments apte à retenir les selles dans le compartiment supérieur tout en permettant leur communication pour l'urine (300), et dans lequel, dans la deuxième configuration, l'obstacle est sensiblement effacé de manière à laisser passer les selles vers le fond de la chambre (20), l'article étant **caractérisé en ce que**, dans la première configuration, la communication entre les compartiments comprend une ouverture (20B ; 120B) sensiblement en forme de fente, la fente étant délimitée par deux plis rentrants (23L, 23R ; 123L, 123R) des deux parties de film souple (23A, 23B ; 123A, 123B) placées l'un en face de l'autre, et l'obstacle étant formé par les deux plis rentrants, et **en ce que** les deux parties de film souple (23A, 23B ; 123A, 123B) sont reliées à leur extrémité supérieure au bord de soutien (10A) et à leur extrémité inférieure à la partie de fond (22), de sorte que le passage de l'élément de paroi interne (23 ; 123) de la première configuration à la deuxième configuration peut être provoqué en tirant le bord de soutien (10A) vers le haut dans la direction verticale (Dv) de l'article (1 ; 100).

2. Article selon la revendication 1, dans lequel la fente (20B ; 120B) est sensiblement dans la direction longitudinale (Dℓ) de l'article (1 ; 100) ou sensiblement dans la direction transversale (Dt) de l'article.

3. Article selon la revendication 1 ou 2, dans lequel, dans la première configuration, la largeur de la fente (20B ; 120B) est comprise entre 0,5 cm et 3 cm.

4. Article selon l'une quelconque des revendications 1 à 3, dans lequel la longueur de la fente (20B ; 120B) est comprise entre 10 cm et 60 cm.

5. Article selon l'une quelconque des revendications 1 à 4, dans lequel une partie de la chambre (20A ; 120) s'étend jusque sous le bord de soutien (10A).

6. Article selon l'une quelconque des revendications 1 à 5, dans lequel un élément absorbant (24 ; 124) est disposé sur la partie de fond (22).

7. Article selon l'une quelconque des revendications 1 à 6, dans lequel le moyen d'écartement (10) est un boudin gonflable.

8. Article selon la revendication 7, dans lequel le bord de soutien (10A) est sur la surface supérieure du boudin gonflable (10).

9. Article selon la revendication 7 ou 8, dans lequel le boudin gonflable (10) comprend une valve de gonflage (16) munie d'un clapet anti-retour (16B).

10. Article selon l'une quelconque des revendications 1 à 9, dans lequel la plus grande dimension de l'ouverture (10E) délimitée par le bord de soutien (10A) est au moins égale à 20 cm.

## Patentansprüche

1. Artikel (1; 100) zur Aufnahme von Urin (300) und Stuhl (400) eines Benutzers, umfassend:
einen Stützrand (10A), um das Gesäß des Benutzers zu tragen, und
eine Kammer (20), die mit einer Öffnung (10E) in Verbindung steht, die von dem Stützrand (10A) begrenzt ist, und die ein Bodenteil (22) aufweist, und
ein Abstandsmittel (10), das geeignet ist, das Bodenteil in einem Abstand von dem Stützrand (10A) zu halten,
wobei die Kammer ein Innenwandelement (23; 123) aufweist, das in einer Zwischenposition vertikal zwischen dem Stützrand (10A) und dem Bodenteil (22) angeordnet ist und das geeignet ist, eine erste Konfiguration und eine zweite Konfiguration einzunehmen, wobei das Innenwandelement (23; 123) zwei Teile einer flexiblen Folie (23A, 23B; 123A, 123B) aufweist,
wobei das Innenwandelement (23; 123) in der ersten Konfiguration die Kammer in ein oberes Abteil und ein unteres Abteil unterteilt, indem ein Hindernis zwischen diesen Abteilen ausgebildet wird, das geeignet ist, den Stuhl in dem oberen Abteil zurückzuhalten und dabei gleichzeitig ihre Verbindung für den Urin (300) ermöglicht, und wobei das Hindernis in der zweiten Konfiguration im Wesentlichen derart beseitigt ist, um den Stuhl zu dem Boden der Kammer (20) durchzulassen, wobei der Artikel **dadurch gekennzeichnet ist, dass** die Verbindung zwischen den Abteilen in der ersten Konfiguration eine im Wesentlichen schlitzförmige Öffnung (20B; 120B) aufweist, wobei der Schlitz durch zwei einspringende Falten (23L, 23R; 123L, 123R) der zwei Teile einer flexiblen Folie (23A, 23B; 123A, 123B) begrenzt ist, die einander zugewandt angeordnet sind, und wobei das Hindernis durch die zwei einspringenden Falten gebildet ist, und dadurch, dass die zwei Teile einer flexiblen Folie (23A, 23B; 123A, 123B) an ihrem oberen Ende mit dem Stützrand (10A) und an ihrem unteren Ende mit dem Bodenteil (22) derart verbunden sind, dass der Übergang des Innenwandelements (23; 123) von der ersten Konfiguration in die zweite Konfiguration bewirkt werden kann, indem der Stützrand (10A) nach oben in die vertikale Richtung (Dv) des Artikels (1; 100) gezogen wird.

2. Artikel nach Anspruch 1, wobei der Schlitz (20B; 120B) im Wesentlichen in der Längsrichtung (Dℓ) des Artikels (1; 100) oder im Wesentlichen in der Querrichtung (Dt) des Artikels verläuft.

3. Artikel nach Anspruch 1 oder 2, wobei die Breite des Schlitzes (20B; 120B) in der ersten Konfiguration zwischen 0,5 cm und 3 cm beträgt.

4. Artikel nach einem der Ansprüche 1 bis 3, wobei die Länge des Schlitzes (20B; 120B) zwischen 10 cm und 60 cm beträgt.

5. Artikel nach einem der Ansprüche 1 bis 4, wobei sich ein Teil der Kammer (20A; 120) bis zu dem Stützrand (10A) erstreckt.

6. Artikel nach einem der Ansprüche 1 bis 5, wobei ein Absorptionselement (24; 124) auf dem Bodenteil (22) angeordnet ist.

7. Artikel nach einem der Ansprüche 1 bis 6, wobei das Abstandsmittel (10) ein aufblasbarer Wulst ist.

8. Artikel nach Anspruch 7, wobei sich der Stützrand (10A) auf der Oberseite des aufblasbaren Wulstes (10) befindet.

9. Artikel nach Anspruch 7 oder 8, wobei der aufblasbare Wulst (10) ein Aufblasventil (16) aufweist, das mit einem Rückschlagventil (16B) ausgestattet ist.

10. Artikel nach einem der Ansprüche 1 bis 9, wobei die größte Abmessung der Öffnung (10E), die von dem Stützrand (10A) begrenzt ist, mindestens gleich 20 cm ist.

## Claims

1. An article (1; 100) for collecting urine (300) and stool (400) of a user, the article comprising:
a support surface (10A) for supporting the buttocks of the user; and
a chamber (20) that communicates with an opening (10E) defined by the support surface (10A) and that presents a bottom portion (22); and
spacer means (10) suitable for maintaining the bottom portion at a distance from the support surface;
the chamber comprising an internal wall element (23; 123) that is situated at a position vertically intermediate between the support surface (10A) and the bottom portion (22) and that is suitable for adopting a first configuration and a second configuration, the internal wall element (23;123) comprising two flexible film portions (23A, 23B; 123A, 123B);
wherein, in the first configuration, the internal wall element (23; 123) subdivides the chamber into a top compartment and a bottom compartment, forming an obstacle between these compartments that is suitable for retaining stools in the top compartment while putting the compartments into communication for urine (300), and wherein, in the second configuration, the obstacle is substantially eliminated so as to allow stool to pass into the bottom of the chamber (20);
the article being **characterized in that**, in the first configuration, communication between the compartments comprises an opening (20B; 120B) that is substantially slot-shaped, the slot being defined by two reentrant folds (23L, 23R; 123L, 123R) in the two flexible film portions (23A, 23B; 123A, 123B) placed facing each other, and the obstacle being formed by the two reentrant folds, and **in that** the two flexible film portions (23A, 23B; 123A, 123B) are connected at their top end to the support surface (10A) and at their bottom end to the bottom portion (22), such that passing of the internal wall element (23;123) from the first configuration to the second configuration may be caused by pulling the support surface (10A) upwards in the vertical direction (Dv) of the article (1;100).

2. An article according to claim 1, wherein the slot (20B; 120B) lies substantially in the longitudinal direction (Df) of the article (1; 100), or substantially in the transverse direction (Dt) of the article.

3. An article according to claim 1 or claim 2, wherein, in the first configuration, the width of the slot (20B; 120B) lies in the range 0.5 cm to 3 cm.

4. An article according to any one of claims 4 to 3, wherein the length of the slot (20B; 120B) lies in that range 10 cm to 60 cm.

5. An article according to any one of claims 1 to 4, wherein a portion of the chamber (20A; 120) extends under the support surface (10A).

6. An article according to any one of claims 1 to 5, wherein an absorbent element (24; 124) is placed on the bottom portion (22).

7. An article according to any one of claims 1 to 6, wherein the spacer means (10) are an inflatable tube.

8. An article according to claim 7, wherein the support surface (10A) is on the top surface of the inflatable tube (10).

9. An article according to claim 7 or claim 8, wherein the inflatable tube (10) includes an inflation valve (16) having a check valve (16B).

10. An article according to any one of claims 1 to 9, wherein the long dimension of the opening (10E) defined by the support surface (10A) is not less than 20 cm.
